# EUROPÄISCHE PATENTSCHRIFT

(11) **EP 1 029 586 B1**
(45) Veröffentlichungstag und Bekanntmachung des Hinweises auf die Patenterteilung: **18.05.2005**
(21) Anmeldenummer: 00102086.6
(22) Anmeldetag: 03.02.2000
(51) Int. Cl.: B01F 17/00

(54) **Emulgatoren**
Emulsifiers
Emulsifiants

(30) Priorität: 16.02.1999 DE 19906368
(43) Veröffentlichungstag der Anmeldung: 23.08.2000
(73) Patentinhaber: Clariant GmbH, 65929 Frankfurt am Main (DE)
(72) Erfinder: Turowski-Wanke, Angelika Dr., 65779 Kelkheim (DE); Löffler, Matthias Dr., 65527 Niedernhausen (DE); Scherl, Franz Xaver Dr., 84508 Burgkirchen (DE)

(56) Entgegenhaltungen:
- EP-A- 0 396 406
- US-A- 5 290 479

## Beschreibung

Die Erfindung betrifft kosmetische und pharmazeutische Emulgatoren mit sehr effektiver Wirkung auf die Erniedrigung der Grenzflächenspannung von sowohl polaren als auch unpolaren Flüssigkeiten, guter Hautfreundlichkeit, hoher Stabilität auch bei Temperaturbelastung und bei geringen Einsatzkonzentrationen und raschem Spreitungsvermögen an der Grenzfläche. Die erfindungsgemäßen, niedrig schmelzenden Emulgatoren bzw. Emulgatormischungen auf Basis nachwachsender Rohstoffe zeigen herausragende hydrophile und hydrophobe Eigenschaften, die gute Emulgatorwirkung sowohl in Öl-in-Wasser als auch in Wasser-in-Öl Formulierungen bedingen.

Zur Herstellung von emulgierten Kosmetika sind als Emulgatoren vielfach Kombinationen von Fettsäuresalzen und nichtionischen Tensiden eingesetzt worden. Nachteilig ist dabei die durch die Gegenwart des Fettsäuresalzes bedingte Steigerung des pH-Wertes, wodurch das kosmetische Mittel alkalisch reagiert. Da die Oberfläche der menschlichen Haut mit einer schwach sauren Membran (pH 4,5 bis 6,5) bedeckt ist, wird angestrebt, Kosmetika auf einen schwach sauren oder neutralen pH einzustellen. Durch Zugabe von Neutralisationsmittel (beispielsweise Natrium- oder Kaliumhydroxid, Triethanolamin) wird die emulgierende Wirkung des Systems jedoch beeinträchtigt.

In DE 29 50 917 wird die Verwendung von Mischungen aus Polyoxyethylensorbitolfettsäureestem und Fettsäuresalzen als W/O-Emulgatoren beschrieben.

GB-A-2 146 528 erwähnt die Verwendung von Sorbitolester als Emulgatoren in Präparaten für Haar oder Haut.

DE 197 27 950 beschreibt die Herstellung von Mischungen, die größtenteils aus Sorbitmonoestern, Sorbitdiestem und Partialglyceriden bestehen.

Es wurde nun gefunden, daß Mischungen aus offenkettigen Sorbitmonoestern, -diestem und Partialglyceriden mit geringen Mengen an cyclischen Sorbitesteranteilen eine sehr effektive Wirkung auf die Erniedrigung der Grenzflächenspannung von sowohl polaren, als auch unpolaren Flüssigkeiten haben, eine hohe Stabilität der Emulsionen auch bei Temperaturbelastung bewirken, eine geringe Empfindlichkeit gegenüber Elektrolyten und Säuren zeigen und sehr hautfreundlich sind.

Gegenstand der Erfindung sind Emulgatoren, die erhalten werden durch Umesterung von gegebenenfalls oxalkyliertem Sorbit mit Fettsäuremethylestem oder Fettsäuretriglyceriden oder Oxalkylierung der durch Umesterung mit Fettsäuremethylestern erhaltenen Reaktionsprodukten, wobei die Umesterung bei Temperaturen von 120 bis 140°C in Gegenwart eines alkalischen Katalysators stattfindet, das Molverhältnis von Sorbit zu Fettsäuremethylester 1:1 bis 1:2 beträgt und das Molverhältnis von Sorbit zu Fettsäuretriglycerid 1:3,5 bis 1:4,5 beträgt.

Die erfindungsgemäßen Emulgatoren werden hergestellt durch Reaktion von Sorbit mit Fettsäuremethylestern oder Fettsäuretriglyceriden entsprechend dem in DE 197 27 950 beschriebenen Verfahren, wobei als Katalysatoren hier allerdings übliche alkalische Katalysatoren, insbesondere Natriummethylat eingesetzt werden. Der Fettsäurerest in den Fettsäuremethylestern und Fettsäuretriglyceriden enthält im allgemeinen 8 bis 22 C-Atome und kann geradkettig oder verzweigt, gesättigt oder ungesättigt sein. Beispiele hierfür sind Palmitinsäure, Stearinsäure, Laurinsäure, Linolsäure, Linolensäure, Isostearinsäure oder Ölsäure. Als Fettsäuretriglyceride kommen alle nativen tierischen oder pflanzlichen Öle, Fette und Wachse in Frage, beispielsweise Olivenöl, Rüböl, Palmkernöl, Sonnenblumenöl, Kokosöl, Leinöl, Ricinusöl, Sojabohnenöl, gegebenenfalls auch in raffinierter oder hydrierter Form. Da diese natürlichen Fette, Öle und Wachse normalerweise Mischungen von Fettsäuren mit unterschiedlicher Kettenlänge darstellen, gilt dies auch für die Fettsäurereste in den erfindungsgemäßen Emulgatoren.

Die Umsetzung von Sorbit mit den Fettsäuretriglyceriden oder Methylestern geschieht im Eintopfverfahren ohne Lösemittel bei Temperaturen von 120 - 140°C in Gegenwart eines alkalischen Katalysators. Die Reaktionszeit beträgt im allgemeinen 12 bis 13 Stunden. Bei der Verwendung von Fettsäuremethylester wird während dieser Reaktion das entstehende Methanol abdestilliert. Da Sorbit üblicherweise als wäßrige Lösung im Handel ist, muß zunächst das Wasser entfernt werden. Dies geschieht durch Destillation bei maximal 120°C unter vermindertem Druck. Das Molverhältnis von Sorbit zu Fettsäuremethylester beträgt 1:1 bis 1:2. Bei Verwendung von Fettsäuretriglyceriden beträgt das Molverhältnis Sorbit zu Fettsäuretriglycerid 1:3,5 bis 1:4,5.

Die Umsetzungsprodukte können auch alkoxyliert sein, vorzugsweise ethoxyliert, der Gehalt an Ethoxylatgruppen kann 1 bis 90 -CH₂CH₂O-Gruppen auf ein Molekül Sorbit betragen. Die Einführung der Alkoxylatgruppen kann erfolgen durch eine Alkoxylierung des Sorbits nach an sich bekannten Verfahren vor der Umesterungsreaktion. Bei der Umesterung mit Fettsäuremethylestern arbeitet man jedoch vorzugsweise so, daß man zunächst die Umesterung durchführt und anschließend nach an sich bekannten Verfahren die Alkoxylatgruppen einführt.

Das Reaktionsprodukt dieser Umesterungsreaktion besteht neben Restmengen an nicht umgesetztem Sorbit im wesentlichen aus den Sorbitmonofettsäureestern und den Sorbitdifettsäureestern. Die entsprechenden Triester entstehen nur in untergeordneten Mengen. Bei der Verwendung von Fettsäuretriglyceriden als Ausgangsprodukt enthält das Reaktionsprodukt auch noch Mono- und Di-Fettsäureglycerid sowie nicht umgesetztes Triglycerid in Abhängigkeit von dem jeweils gewählten Molverhältnis der Ausgangsverbindungen.

Die so erhaltenen Mischung der verschiedenen Reaktionsprodukte eignet sich sehr gut als Emulgatoren für alle Arten von pharmazeutischen und kosmetischen Emulsionen sowohl vom Öl-in-Wasser als auch vom Wasser-in-Öl Typ. Die Menge der erfindungsgemäßen Umesterungsprodukte als Emulgator in diesen Emulsionen beträgt im allgemeinen 0,1 bis 8, bevorzugt 0,3 bis 5, insbesondere 0,5 bis 4 Gew.-%, bezogen auf die Emulsion.

Die erfindungsgemäßen Produkte lassen sich durch Auswahl der Fettsäurekomponente, durch den Ethoxylierungsgrad und/oder durch Zusatz von Neutralisationsmittel, aber auch durch Zusatz von hydrophilen Komponenten, beispielsweise Acylglutamate, Alkylpolyglycoside, Isethionate, Ascorbinsäure und Coemulgatoren, beispielsweise Hostacerin® DGI, Hostacerin® DGL, in einem sehr weiten Hydrophilie-Bereich herstellen. Es ist dadurch möglich, geeignete Emulgatoren für zahlreiche Öle, Fette, Wachse, Paraffine, für nicht wassermischbare Lösungsmittel und Wirkstoffe im kosmetischen und pharmazeutischen Sektor bereitzustellen.

Der nichtwäßrige Anteil der Emulsionen, der sich weitgehend aus dem Emulgator- und dem Ölkörper zusammensetzt liegt üblicherweise bei 5 bis 95 % und vorzugsweise 15 bis 75 Gew.-%. Das bedeutet, daß die Emulsionen 5 bis 95 und vorzugsweise 25 bis 85 Gew.-% Wasser enthalten können, abhängig davon, ob Lotionen mit einer vergleichsweise niedrigen oder Cremes und Salben mit hoher Viskosität hergestellt werden sollen.

Als Ölkörper kommen beispielsweise Guerbetalkohole mit 6 bis 18, vorzugsweise 8 bis 10 Kohlenstoffatomen, Ester von linearen C₆-C₁₃-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimerdiol) und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate, Dialkylether und/oder aliphatische bzw. aromatische Kohlenwasserstoffe in Betracht.

Die Emulsionen können als Hautpflegemittel, wie beispielsweise Tagescremes, Nachtcremes, Pflegecremes, Nährcreme, Bodylotions, Salben und dergleichen vorliegen und als weitere Hilfs- und Zusatzstoffe, Co-Emulgatoren, Uberfettungsmittel, Fette, Wachse, Stabilisatoren, biogene Wirkstoffe, Glycerin, Konservierungsmittel, Perlglanzmittel, Farb- und Duftstoffe enthalten.

Als nichtionogene O/W-Co-Emulgatoren kommen in Betracht Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe; C₁₂-C₁₈-Fettsäuremono- und -diester von Anlagerungsprodukten von 1 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester wie z.B. Polyglycerinpolyricinoleat und Polyglycerinpoly-12-hydroxystearat. Ebenfalls geeignet sind Gemische von Verbindungen aus mehreren dieser Substanzklassen. Die Anlagerungsprodukte von Ethylenoxid und/oder Propylenoxid an Fettalkohole, Fettsäuren, Alkylphenole, Glycerin-mono- und -diester sowie Sorbitanmono- und -diester von Fettsäuren oder an Rizinusöl stellen bekannte, im Handel erhältliche Produkte dar. Es handelt sich dabei um Homologengemische, deren mittlerer Alkoxilierungsgrad dem Verhältnis der Stoffmengen von Ethylenoxid und/oder Propylenoxid und Substrat, mit denen die Anlagerungsreaktion durchgeführt wird, entspricht. C₁₂C₁₈-Fettsäuremono- und -diester von Anlagerungsprodukten von Ethylenoxid an Glycerin sind aus DE-20 24 051 als Rückfettungsmittel für kosmetische Zubereitungen bekannt.

Als Überfettungsmittel können Substanzen wie beispielsweise polyethoxilierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Typische Beispiele für Fette sind Glyceride, als Wachse kommen u.a. Bienenwachs, Paraffinwachs oder Mikrowachse, gegebenenfalls in Kombination mit hydrophilen Wachsen, z.B. Cetylstearylalkohol in Frage. Als Stabilisatoren können Metallsalze von Fettsäuren, wie z.B. Magnesium-, Aluminium- und/oder Zinkstearat eingesetzt werden. Unter biogenen Wirkstoffen sind beispielsweise Pflanzenextrakte und Vitaminkomplexe zu verstehen. Als Konservierungsmittel eignen sich beispielsweise Phenoxyethanol, Formaldehydlösung, Parabene, Pentandiol oder Sorbinsäure. Als Periglanzmittel kommen beispielsweise Glycoldistearinsäureester wie Ethylenglycoldistearat, aber auch Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S. 81 - 106, zusammengestellt sind.

Der Gesamtanteil der Hilfs- und Zusatzstoffe kann 1 bis 10, vorzugsweise 2 bis 5 Gew.-%, bezogen auf das Mittel, betragen.
Die Herstellung der Mittel kann in an sich bekannter Weise, d.h. beispielsweise durch Heiß-, Heiß-Heiß/Kalt- bzw. PIT-Emulgierung erfolgen.

### Beispiele

### Sorbitester 1

1 Mol Sorbit in Form eines 70 %igen Sorbitsirups wurden vorgelegt und das Wasser im Wasserstrahlvakuum bei 120°C abdestilliert. Dann wurden 1 Gew.-% (bezogen auf die gesamte Einwaage) NaOCH₃ (30 %ig in Methanol) bei 80°C zugegeben und bei vollem Wasserstrahlvakuum das Methanol abdestilliert. Dann wurden 2 Mol Laurinsäuremethylester zugegeben und 1 Stunde bei 140°C unter Stickstoff und weiterhin 12 Stunden bei 140°C und vollem Wasserstrahlvakuum erhitzt. Das entstandene Methanol wurde abdestilliert.

### Sorbitester 2

Arbeitsweise wie bei Sorbitester 1. Die Umesterung erfolgte durch Erhitzen über 11 Stunden bei 140°C mit 1 Mol Palmitinsäuremethylester.

### Sorbitester 3

1 Mol Sorbit in Form eines 70 %igen Sorbitsirups wurden vorgelegt und das Wasser im Wasserstrahlvakuum bei 120°C abdestilliert. Nach Zugabe von 1 Gew.-% (bezogen auf die gesamte Einwaage) Kaliumcarbonat (30 %ig in Wasser) bei 80°C wurde bei vollem Wasserstrahlvakuum das Wasser abdestilliert. Anschließend wurden 4 Mol raffiniertes Rapsöl zugegeben und 8 Stunden bei 140°C gerührt.

Die nachfolgenden Beispiele sollen den Erfindungsgegenstand näher erläutern, ohne ihn darauf zu beschränken.

### Anwendungsbeispiele

### 1. O/W-Creme

| | | |
|---|---|---|
| A | Sorbitester (Reaktionsgemisch) gemäß Beispiel 1 | 5,00 % |
| | Paraffinöl, niedrigviskos | 10,00 % |
| | ®Eutanol G | 7,00 % |
| | Mandelöl | 6,00 % |
| | Antioxidantien | q.s. |
| | | |
| B | ®Carbopol 980 | 0,50 % |
| C | ®Hostapon KCG | 0,40 % |
| | NaOH (10 %ig) | 2,00 % |
| | ®Aquamollin BC PIv. Hochkonz. | 0,10 % |
| | Citronensäure (10 %ig) | 0,25 % |
| | Wasser | ad 100 % |
| | | q.s. |
| D | Parfümöl | 0,40 % |

### Herstellung

| | |
|---|---|
| I | A bei ca. 80°C aufschmelzen, anschließend B eintragen |
| II | C auf ca. 80°C erwärmen |
| III | II unter Rühren I zusetzen und kaltrühren |
| IV | bei ca. 35°C D in III einrühren |
| V | abschließend die Emulsion homogenisieren |

### 2. O/W-Creme

| | | |
|---|---|---|
| A | Sorbitester (Reaktionsgemisch) gemäß Beispiel 1 | 2,00 % |
| | Paraffinöl, perliquidum | 8,00 % |
| | ®Eutanol G | 4,00 % |
| | Isopropylpalmitat | 4,00 % |
| B | ®Carbopol 980 | 0,70 % |
| C | ®Hostapon KCG | 0,80 % |
| | Wasser | ad 100 % |
| | Natronlauge (10 %ig) | 2,80 % |
| | Konservierungsmittel | q.s. |
| | | |
| D | Parfümöl | 0,40 % |

### Herstellung

| | |
|---|---|
| I | A und B bei Raumtemperatur mischen |
| II | C in I einrühren |
| III | D in II einrühren |
| IV | abschließend die Emulsion homogenisieren |

### 3. O/W-Hautmilch, mineralölfrei

| | | |
|---|---|---|
| A | Sorbitester (Reaktionsgemisch) gemäß Beispiel 2 | 2,00 % |
| | Isopropylpalmitat | 6,00 % |
| | Avocadoöl | 7,00 % |
| | Mandelöl | 7,00 % |
| | | |
| B | Carbopol 980 | 0,30 % |
| C | Hostapon KCG | 0,60 % |
| | NaOH (10 %ig) | 1,20 % |
| | ®Aquamollin BC PIv. Hochkonz. | 0,10 % |
| | Polyethylenglykol 400 | 4,00 % |
| | Wasser | ad 100 % |
| | Konservierungsmittel | q.s. |
| | | |
| D | Parfümöl | 0,30 % |

### Herstellung

| | |
|---|---|
| I | A bei ca. 80°C aufschmelzen, anschließend B eintragen |
| II | C auf ca. 80°C erwärmen |
| III | II unter Rühren I zusetzen und kaltrühren |
| IV | bei ca. 35°C D in III einrühren |
| V | abschließend die Emulsion homogenisieren |

### 4. O/W-Reinigungsmilch

| | | |
|---|---|---|
| A | Sorbitester (Reaktionsgemisch) gemäß Beispiel 2 | 2,00 % |
| | Cetylalkohol | 1,50 % |
| | Paraffinöl, hochviskos | 15,00 % |
| | ®Cetiol SN | 8,00 % |
| | ®Solulan 98 | 2,00 % |
| | | |
| B | Carbopol 980 | 0,30 % |
| C | Hostapon KCG | 0,80 % |
| | NaOH (10 %ig) | 1,20 % |
| | Wasser | ad 100 % |
| | Konservierungsmittel | q.s. |
| | | |
| D | Parfümöl | 0,40 % |

### Herstellung

| | |
|---|---|
| I | A bei ca. 60°C aufschmelzen, anschließend B eintragen |
| II | C auf ca. 60°C erwärmen |
| III | II unter Rühren I zusetzen und kaltrühren |
| IV | bei ca. 35°C D in III einrühren |
| V | abschließend die Emulsion homogenisieren |

### 5. Wasser-in-Öl-Creme

### Zusammensetzung

| | | |
|---|---|---|
| A | Sorbitester (Reaktionsgemisch) gemäß Beispiel 3 | 4,00 % |
| | Bienenwachs | 2,00 % |
| | ®Lunacera M | 3,00 % |
| | Paraffinöl | 12,50 % |
| | Isopropylpalmitat | 7,50 % |
| | Sojaöl | 5,00 % |
| | Magnesiumstearat | 1,00 % |
| | | |
| B | Wasser | ad 100 % |

### Herstellung

| | |
|---|---|
| I | Phase A auf 80°C erwärmen |
| II | Wasser auf 80°C erwärmen |
| III | Wasser unter Rühren zu Phase A geben |
| IV | entstandene Emulsion kaltrühren |
| V | Emulsion homogenisieren |

### 6. Wasser-in-Öl-Creme

### Zusammensetzung

| | | |
|---|---|---|
| A | Sorbitester (Reaktionsgemisch) gemäß Beispiel 3 | 3,50 % |
| | ®Hostacerin DGI | 1,50 % |
| | Bienenwachs | 2,00 % |
| | Lunacera M | 3,00 % |
| | Mandelöl | 5,00 % |
| | Avocadoöl | ..5,00 % |
| | Sojaöl | 12,50 % |
| | Jojobaöl | 2,50 % |
| | Magnesiumstearat | 1,00 % |
| | | |
| B | Wasser | ad 100 % |

### Herstellung

| | |
|---|---|
| I | Phase A auf 80°C erwärmen |
| II | Wasser auf 80°C erwärmen |
| III | Wasser unter Rühren zu Phase A geben |
| IV | entstandene Emulsion kaltrühren |
| V | Emulsion homogenisieren |

Alle Prozentangaben in den Anwendungsbeispielen 1 bis 6 sind Gewichtsprozente.

### Verzeichnis der eingesetzten Produkte

| | |
|---|---|
| Paraffinöl, niedrigviskos | Mineralöl |
| Paraffinöl perliquidum | Mineralöl |
| Eutanol G (Henkel KGaA) | Octyldodecanol |
| Carbopol 980 (Goodrich) | vernetzte Polyacrylsäure |
| Hostapon KCG (Clariant GmbH) | Natriumcocoylglutamat |
| Aquamollin BC PIv (Clariant GmbH) | Ethylendiamintetraacetat, Na-Salz |
| Cetiol SN (Henkel KGaA) | Cetearylisononanoat |
| Solulan 98 (Henkel KGaA) | Mischung aus Polysorbat, Cetylacetat und acetyliertem Lanolinalkohol |
| Lunacera M | Mikrokristallines Wachs |
| Hostacerin DGI (Clariant GmbH) | Fettsäurepolyglycerinester |

## Patentansprüche

1. Emulgatoren, erhalten durch Umesterung von gegebenenfalls oxalkyliertem Sorbit mit Fettsäuremethylestern oder Fettsäuretriglyceriden oder Oxalkylierung der durch Umesterung mit Fettsäuremethylestern erhaltenen Reaktionsprodukte, **dadurch gekennzeichnet, dass** die Umesterung bei Temperaturen von 120 bis 140°C in Gegenwart eines alkalischen Katalysators stattfindet, das Molverhältnis von Sorbit zu Fettsäuremethylester 1:1 bis 1:2 beträgt und das Molverhältnis von Sorbit zu Fettsäuretriglycerid 1:3,5 bis 1:4,5 beträgt.

2. Emulgatoren nach Anspruch 1, erhalten durch Umesterung von Sorbit mit Fettsäuremethylestern oder Fettsäuretriglyceriden, **dadurch gekennzeichnet, dass** die Umesterung bei Temperaturen von 120 bis 140°C in Gegenwart eines alkalischen Katalysators stattfindet, das Molverhältnis von Sorbit zu Fettsäuremethylester 1:1 bis 1:2 beträgt und das Molverhältnis von Sorbit zu Fettsäuretriglycerid 1:3,5 bis 1:4,5 beträgt.

3. Emulgatoren nach Anspruch 1 oder 2, **dadurch gekennzeichnet, dass** die Fettsäure in den Fettsäuremethylestern oder Fettsäuretriglyceriden 8 bis 22 C-Atome enthält.

4. Emulgatoren nach Anspruch 1 oder 3, **dadurch gekennzeichnet, dass** sie 1 bis 90 Ethoxylatgruppen pro Molekül Sorbit enthalten.

5. Pharmazeutische oder kosmetische Emulsionen enthaltend einen Emulgatoren nach einem oder mehreren der Ansprüche 1 bis 4.

6. Pharmazeutische oder kosmetische Emulsionen nach Anspruch 5, enthaltend 0,1 bis 8 Gew.-% eines Emulgators nach einem oder mehreren der Ansprüche 1 bis 4.

7. Verfahren zur Herstellung von Emulgatoren, **dadurch gekennzeichnet, dass** man gegebenenfalls oxalkylierten Sorbit mit Fettsäuremethylestern oder Fettsäuretriglyceriden umestert oder das durch Umesterung von Fettsäuremethylestern mit Sorbit erhaltene Reaktionsprodukt oxalkyliert, die Umesterung bei Temperaturen von 120 bis 140°C in Gegenwart eines alkalischen Katalysators stattfindet, das Molverhältnis von Sorbit zu Fettsäuremethylester 1:1 bis 1:2 beträgt und das Molverhältnis von Sorbit zu Fettsäuretriglycerid 1:3,5 bis 1:4,5 beträgt.

8. Verfahren zur Herstellung von Emulgatoren nach Anspruch 7, **dadurch gekennzeichnet, dass** man Sorbit mit Fettsäuremethylestern oder Fettsäuretriglyceriden umestert, die Umesterung bei Temperaturen von 120 bis 140°C in Gegenwart eines alkalischen Katalysators stattfindet, das Molverhältnis von Sorbit zu Fettsäuremethylester 1:1 bis 1:2 beträgt und das Molverhältnis von Sorbit zu Fettsäuretriglycerid 1:3,5 bis 1:4,5 beträgt.

## Claims

1. An emulsifier obtained by transesterification of optionally alkoxylated sorbitol with fatty acid methyl esters or fatty acid triglycerides, or alkoxylation of the reaction products obtained by transesterification with fatty acid methyl esters, wherein the transesterification takes place at temperatures of from 120 to 140°C in the presence of an alkaline catalyst, the molar ratio of sorbitol to fatty acid methyl ester is from 1:1 to 1:2, and the molar ratio of sorbitol to fatty acid triglyceride is from 1:3.5 to 1:4.5.

2. An emulsifier as claimed in claim 1, obtained by transesterification of sorbitol with fatty acid methyl esters or fatty acid triglycerides, wherein the transesterification takes place at temperatures of from 120 to 140°C in the presence of an alkaline catalyst, the molar ratio of sorbitol to fatty acid methyl ester is from 1:1 to 1:2, and the molar ratio of sorbitol to fatty acid triglyceride is from 1:3.5 to 1:4.5.

3. An emulsifier as claimed in claim 1 or 2, wherein the fatty acid in the fatty acid methyl esters or fatty acid triglycerides contains from 8 to 22 carbon atoms.

4. An emulsifier as claimed in claim 1 or 3, which contains from 1 to 90 ethoxylate groups per molecule of sorbitol.

5. A pharmaceutical or cosmetic emulsion comprising an emulsifier as claimed in one of more of claims 1 to 4.

6. A pharmaceutical or cosmetic emulsion as claimed in claim 5 comprising from 0.1 to 8% by weight of an emulsifier as claimed in one or more of claims 1 to 4.

7. A process for the preparation of emulsifiers, which comprises transesterifying optionally alkoxylated sorbitol with fatty acid methyl esters or fatty acid triglycerides, or alkoxylating the reaction product obtained by transesterification of fatty acid methyl esters with sorbitol, where the transesterification takes place at temperatures of from 120 to 140°C in the presence of an alkaline catalyst, the molar ratio of sorbitol to fatty acid methyl ester is from 1:1 to 1:2, and the molar ratio of sorbitol to fatty acid triglyceride is from 1:3.5 to 1:4.5.

8. A process for the preparation of emulsifiers as claimed in claim 7, which comprises transesterifying sorbitol with fatty acid methyl esters or fatty acid triglycerides, where the transesterification takes place at temperatures of from 120 to 140°C in the presence of an alkaline catalyst, the molar ratio of sorbitol to fatty acid methyl ester is from 1:1 to 1:2, and the molar ratio of sorbitol to fatty acid triglyceride is from 1:3.5 to 1:4.5.

## Revendications

1. Emulsifiants obtenus par transestérification du sorbitol éventuellement oxyalkylé par des esters méthyliques d'acides gras ou triglycérides d'acides gras, ou par oxyalkylation des produits réactionnels obtenus par transestérification par des esters méthyliques d'acides gras, **caractérisés en ce que** la transestérification est mise en oeuvre à une température de 120 à 140°C en présence d'un catalyseur alcalin, le rapport molaire du sorbitol à l'ester méthylique d'acide gras est de 1:1 à 1:2 et le rapport molaire du sorbitol au triglycéride d'acide gras est de 1:3,5 à 1:4,5.

2. Emulsifiants selon la revendication 1, obtenus par transestérification du sorbitol par des esters méthyliques d'acides gras ou triglycérides d'acides gras, **caractérisés en ce que** la transestérification est mise en oeuvre à une température de 120 à 140°C en présence d'un catalyseur alcalin, le rapport molaire du sorbitol à l'ester méthylique d'acide gras est de 1:1 à 1:2, et le rapport molaire du sorbitol au triglycéride d'acide gras est de 1:3,5 à 1:4,5.

3. Emulsifiants selon la revendication 1 ou 2, **caractérisés en ce que** l'acide gras dans les esters méthyliques d'acides gras ou les triglycérides d'acides gras présente de 8 à 22 atomes de carbone.

4. Emulsifiants selon la revendication 1 ou 3, **caractérisés en ce qu'**ils renferment de 1 à 90 groupes éthoxylate par molécule de sorbitol.

5. Emulsions pharmaceutiques ou cosmétiques renfermant un émulsifiant selon une ou plusieurs des revendications 1 à 4.

6. Emulsions pharmaceutiques ou cosmétiques selon la revendication 5, renfermant de 0,1 à 8 % en masse d'un émulsifiant selon une ou plusieurs des revendications 1 à 4.

7. Procédé pour la préparation d'émulsifiants, **caractérisé en ce que** le sorbitol éventuellement oxyalkylé est transestérifié par des esters méthyliques d'acides gras ou triglycérides d'acides gras, ou le produit réactionnel obtenu par transestérification d'esters méthyliques d'acides gras par le sorbitol est oxyalkylé, la transestérification est mise en oeuvre à une température de 120 à 140°C en présence d'un catalyseur alcalin, le rapport molaire du sorbitol à l'ester méthylique d'acide gras est de 1:1 à 1:2, et le rapport molaire du sorbitol au triglycéride d'acide gras est de 1:3,5 à 1:4,5.

8. Procédé pour la préparation d'émulsifiants selon la revendication 7, **caractérisé en ce que** le sorbitol est transestérifié par des esters méthyliques d'acides gras ou triglycérides d'acides gras, la transestérification est mise en oeuvre à une température de 120 à 140°C en présence d'un catalyseur alcalin, le rapport molaire du sorbitol à l'ester méthylique d'acide gras est de 1:1 à 1:2, et le rapport molaire du sorbitol au triglycéride d'acide gras est de 1:3,5 à 1:4,5.
